# EUROPEAN PATENT APPLICATION

(11) **EP 4 434 485 A1**
(43) Date of publication of application: **25.09.2024**
(21) Application number: 22895633.0
(22) Date of filing: 16.11.2022
(51) Int. Cl.: A61B 34/37, B25J 3/00

(54) **SURGERY SUPPORT SYSTEM AND SURGERY SUPPORT SYSTEM CONTROL METHOD**

(30) Priority: 19.11.2021 JP 2021188955
(71) Applicant: KAWASAKI JUKOGYO KABUSHIKI KAISHA, Kobe-shi, Hyogo 650-8670 (JP)
(72) Inventor: MIZOHATA, Yuichi, Hyogo 650-8670 (JP)
(74) Representative: Hoffmann Eitle
(86) International application number: PCT/JP2022/042518
(87) International publication number: WO 2023/090352

(57) **Abstract**

A robotic surgical system (100) includes a control device (140) configured or programmed to cause a storage (35) to store, as a second pivot position (PP2), a pivot position after a surgical instrument (4) is moved, when a pivot position setter (85) is operated after the surgical instrument (4) is moved by an operation unit (80) in a pivot position change mode.

## Description

### Technical Field

The present disclosure relates to a robotic surgical system and a control method for a robotic surgical system.

### Background Art

Conventionally, a robotic surgical system including a robot arm to which a surgical instrument is attached is known. Japanese Patent Laid-Open No. 2018-094446 discloses a robotic system including a manipulator arm and a tool attached to the manipulator arm. The manipulator arm disclosed in Japanese Patent Laid-Open No. 2018-094446 includes an instrument holder to translate the tool along a longitudinal direction. A cannula is held at a distal end of the instrument holder. The cannula is held by a cannula holding mechanism of the instrument holder. The tool is inserted into the cannula. In Japanese Patent Laid-Open No. 2018-094446, a predetermined portion of the cannula is predetermined as a fulcrum for rotational movement of the tool. That is, in Japanese Patent Laid-Open No. 2018-094446, the fulcrum for rotational movement of the tool is mechanically determined. Furthermore, Japanese Patent Laid-Open No. 2018-094446 describes that the manipulator arm is reconfigured during surgery in order to avoid interference and expand the range of motion of instruments within a patient.

### Prior Art

### Patent Document

Patent Document 1: Japanese Patent Laid-Open No. 2018-094446

### Summary of the Invention

In Japanese Patent Laid-Open No. 2018-094446, the manipulator arm is reconfigured during surgery with an intermediate portion of the tool constrained to the fulcrum for rotational movement, and the manipulator arm is reconfigured during surgery with an end effector of the tool maintained in a desired state. In Japanese Patent Laid-Open No. 2018-094446, the manipulator arm is used in which the fulcrum for rotational movement of the tool is structurally determined. Therefore, the fulcrum for rotational movement of the tool is set at a predetermined position of the cannula simply by holding the cannula by the cannula holding mechanism of the instrument holder of the manipulator arm. However, the manipulator arm is connected to the cannula through the cannula holding mechanism, and thus a space in the vicinity of the body surface of the patient into which the cannula is inserted becomes narrow. Consequently, when an assistant doctor performs auxiliary work during surgery, the cannula holding mechanism interferes with the work, and it becomes difficult to work in the vicinity of the body surface of the patient.

The present disclosure is intended to solve the above problem. The present disclosure aims to provide a robotic surgical system and a control method for a robotic surgical system that each allow work to be easily performed in the vicinity of the body surface of a patient during surgery, and allow operations to be easily performed to avoid interference of a robot arm and to expand the range of motion of a surgical instrument within the patient.

A robotic surgical system according to a first aspect of the present disclosure includes a robot arm having a distal end to which a surgical instrument is attached, an operation unit attached to the robot arm to operate the robot arm, a pivot position setter to set a pivot position that serves as a fulcrum for movement of the surgical instrument attached to the robot arm, a storage, and a control device. The control device is configured or programmed to cause the storage to store a first pivot position based on the pivot position setter being operated, make a transition to a pivot position change mode for changing the first pivot position stored in the storage based on the pivot position setter being operated in a state in which the surgical instrument is inserted into a body of a patient, and cause the storage to store, as a second pivot position, the pivot position after the surgical instrument is moved, when the pivot position setter is operated after the surgical instrument is moved by the operation unit in the pivot position change mode.

In the surgical robot according to the first aspect of the present disclosure, as described above, the control device is configured or programmed to cause the storage to store the first pivot position based on the pivot position setter being operated. Accordingly, the control device causes the storage to store the pivot position by software, and thus it is not necessary to use a manipulator arm with a structurally defined first pivot position or to arrange a mechanism on a manipulator arm to support a cannula in order to set the first pivot position. Consequently, no mechanism for supporting the cannula is arranged, and thus work in the vicinity of the body surface of the patient can be easily performed during surgery. Furthermore, the control device is configured or programmed to make a transition to the pivot position change mode for changing the first pivot position stored in the storage based on the pivot position setter being operated in a state in which the surgical instrument is inserted into the body of the patient, and to cause the storage to store, as the second pivot position, the pivot position after the surgical instrument is moved, when the pivot position setter is operated after the surgical instrument is moved by the operation unit in the pivot position change mode. Thus, the control device changes the pivot position by software, and thus the first pivot position that serves as the fulcrum for movement of the surgical instrument can be easily changed. Consequently, an operation to avoid interference of the robot arm and an operation to expand the range of motion of the surgical instrument within the patient can be easily performed.

A control method for a robotic surgical system according to a second aspect of the present disclosure includes causing a storage to store a first pivot position that serves as a fulcrum for movement of a surgical instrument attached to a robot arm based on a pivot position setter being operated, making a transition to a pivot position change mode for changing the first pivot position stored in the storage based on the pivot position setter being operated in a state in which the surgical instrument is inserted into a body of a patient, and causing the storage to store, as a second pivot position, a pivot position after the surgical instrument is moved, when the pivot position setter is operated after the surgical instrument is moved by an operation unit in the pivot position change mode.

As described above, the control method for a robotic surgical system according to the second aspect of the present disclosure includes causing the storage to store the first pivot position that serves as the fulcrum for movement of the surgical instrument attached to the robot arm based on the pivot position setter being operated. Accordingly, the control device causes the storage to store the pivot position by software, and thus it is not necessary to use a manipulator arm with a structurally defined first pivot position or to arrange a mechanism on a manipulator arm to support a cannula in order to set the first pivot position. Consequently, no mechanism for supporting the cannula is arranged, and thus it is possible to provide the control method for the robotic surgical system that allows work to be easily performed in the vicinity of the body surface of the patient during surgery. Furthermore, the control method for a robotic surgical system includes making a transition to the pivot position change mode for changing the first pivot position stored in the storage based on the pivot position setter being operated in a state in which the surgical instrument is inserted into the body of the patient, and causing the storage to store, as the second pivot position, the pivot position after the surgical instrument is moved, when the pivot position setter is operated after the surgical instrument is moved by the operation unit in the pivot position change mode. Thus, the control device changes the first pivot position by software, and thus the first pivot position that serves as the fulcrum for movement of the surgical instrument can be easily changed. Consequently, it is possible to provide the control method for a robotic surgical system that allows operations to be easily performed to avoid interference of the robot arm and to expand the range of motion of the surgical instrument within the patient.

According to the present disclosure, it is possible to easily perform work in the vicinity of the body surface of the patient during surgery, and easily perform an operation to avoid interference of the robot arm and an operation to expand the range of motion of the surgical instrument within the patient.

### Brief Description of the Drawings

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing a pair of forceps.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.

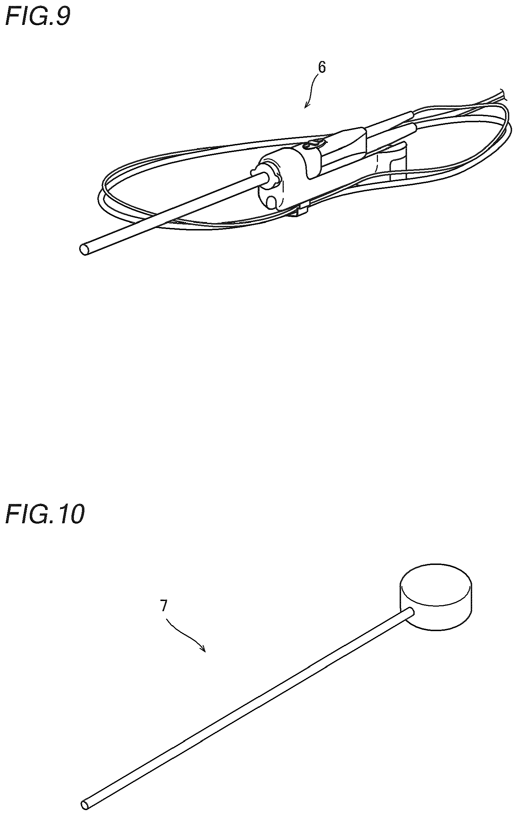

FIG. 9 is a diagram showing an endoscope.
FIG. 10 is a diagram showing a pivot position setting instrument.
FIG. 11 is a control block diagram of a surgical robot according to the embodiment.
FIG. 12 is a control block diagram of the robot arm according to the embodiment.
FIG. 13 is a diagram for illustrating operation to store a pivot position in a storage.
FIG. 14 is a diagram for illustrating changing the pivot position stored in the storage.
FIG. 15 is a diagram for illustrating a pivot correction possible range and a pivot correction start possible range.
FIG. 16 is a diagram for illustrating a pivot deviation monitoring range before the pivot position is changed.
FIG. 17 is a diagram for illustrating the pivot deviation monitoring range after the pivot position is changed.
FIG. 18 is a diagram showing a message displayed on a monitor when the pivot position is outside the pivot correction start possible range.
FIG. 19 is a diagram showing a message displayed on the monitor when the pivot position is located between the pivot correction possible range and the pivot correction start possible range.
FIG. 20 is a diagram for illustrating mode transition.
FIG. 21 is a diagram for illustrating a method for calculating a temporary pivot position.
FIG. 22 is a diagram for illustrating a method for calculating a pivot position after change.
FIG. 23 is a flowchart for illustrating a control method for the surgical robot according to the embodiment. Modes for Carrying Out the Invention

### Configuration of Robotic Surgical System

The configuration of a robotic surgical system 100 according to the present embodiment is now described. The robotic surgical system 100 includes a surgical robot 1 and a remote control apparatus 2.

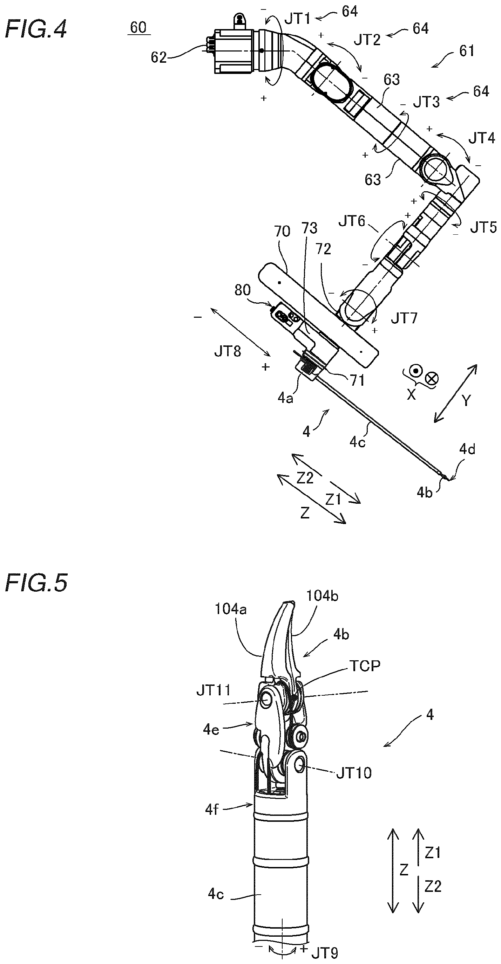

In this description, as shown in FIGS. 4 to 7, the longitudinal direction of a surgical instrument 4 is defined as a Z direction. The distal end side of the surgical instrument 4 is defined as a Z1 side, and the proximal end side of the surgical instrument 4 is defined as a Z2 side. A direction perpendicular to the Z direction is defined as an X direction. One side in the X direction is defined as an X1 side, and the other side in the X direction is defined as an X2 side. A direction perpendicular to the Z direction and the X direction is defined as a Y direction. One side in the Y direction is defined as a Y1 side, and the other side in the Y direction is defined as a Y2 side.

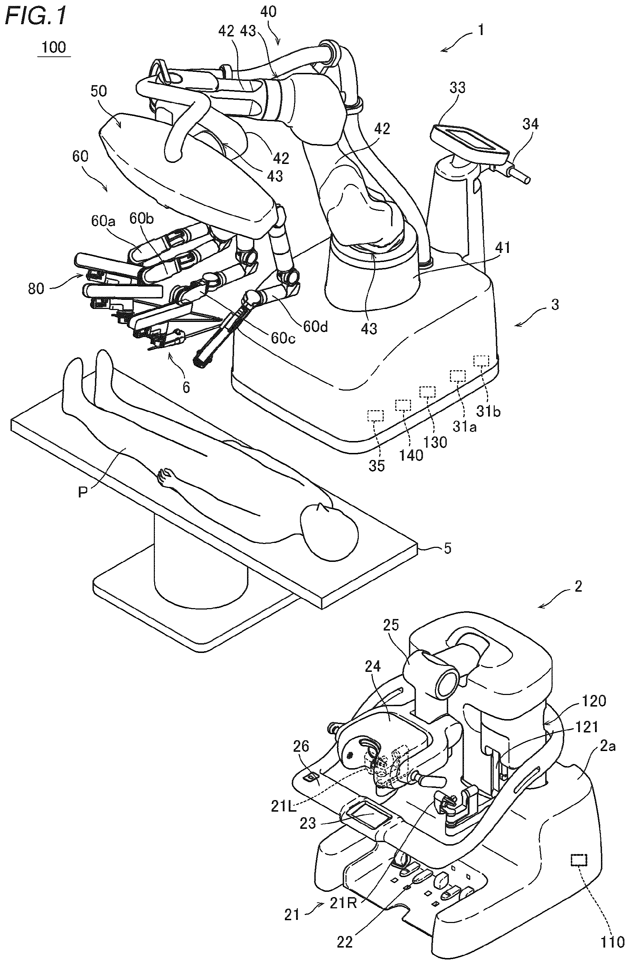

As shown in FIG. 1, the surgical robot 1 is arranged in an operating room. The remote control apparatus 2 is spaced apart from the surgical robot 1. An operator such as a doctor inputs a command to the remote control apparatus 2 to cause the surgical robot 1 to perform a desired operation. The remote control apparatus 2 transmits the input command to the surgical robot 1. The surgical robot 1 operates based on the received command. The surgical robot 1 is arranged in the operating room that is a sterilized sterile field.

### Configuration of Surgical Robot

As shown in FIG. 1, the surgical robot 1 includes a medical cart 3, a positioner 40, an arm base 50, a plurality of robot arms 60, and arm operation units 80. The arm operation units 80 are examples of an operation unit.

The medical cart 3 moves the positioner 40. The medical cart 3 includes an input 33. The input 33 receives operations to move the positioner 40, the arm base 50, and the plurality of robot arms 60 or change their postures mainly in order to prepare for surgery before the surgery. The medical cart 3 includes an operation handle 34 to receive an operator's steering operation.

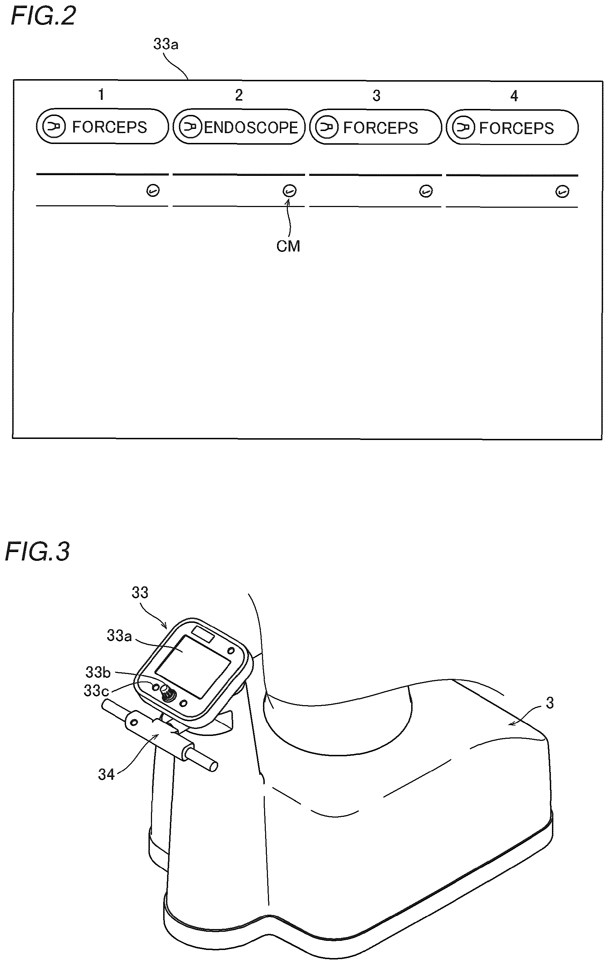

As shown in FIG. 2, a display 33a is provided on the input 33. The display 33a is a liquid crystal panel, for example. Numbers corresponding to the plurality of robot arms 60 are displayed on the display 33a. Furthermore, the type of surgical instrument 4 attached to each of the plurality of robot arms 60 is displayed on the display 33a. A check mark CM indicating that a pivot position PP described below has been set is displayed on the display 33a.

As shown in FIG 3, a joystick 33b for operating movement of the positioner 40 is arranged in the vicinity of the input 33 of the medical cart 3. The positioner 40 is three-dimensionally moved by selecting an operation mode displayed on the input 33 and operating the joystick 33b. The joystick 33b is an example of a first enable switch.

In the vicinity of the joystick 33b of the medical cart 3, an enable switch 33c is provided to enable or disable movement of the positioner 40. The joystick 33b is operated while the enable switch 33c is being pressed to enable movement of the positioner 40 such that the positioner 40 is moved.

As shown in FIG. 1, the positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are coupled to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports the surgical instrument 4.

The plurality of robot arms 60 are arranged. Specifically, four robot arms 60a, 60b, 60c, and 60d are arranged. The robot arms 60a, 60b, 60c, and 60d have the same or similar configurations as each other.

As shown in FIG. 4, each robot arm 60 includes an arm portion 61, a first link 72, a second link 73, and a translation mechanism 70. The robot arm 60 includes JT1 to JT7 axes as rotation axes and a JT8 axis as a linear motion axis. The JT1 to JT7 axes are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along the Z direction.

The arm portion 61 includes a 7-axis articulated robot arm. The first link 72 is arranged at a distal end of the arm portion 61. An arm operation unit 80 described below is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument or an endoscope 6 to capture an image of a surgical site, for example. The surgical instrument 4 as the instrument includes a driven unit 4a, a pair of forceps 4b, and a shaft 4c.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other. The robot arms 60a, 60b, and 60d are examples of a first robot arm. The robot arm 60c is an example of a second robot arm.

### Configuration of Instrument

As shown in FIG. 5, the pair of forceps 4b is provided at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

The pair of forceps 4b includes a first support 4e that supports the proximal end sides of jaw members 104a and 104b such that the proximal end sides of the jaw members 104a and 104b are rotatable about a JT11 axis on the distal end sides, and a second support 4f that supports the proximal end side of the first support 4e such that the proximal end side of the first support 4e is rotatable about a JT10 axis on the distal end side. The shaft 4c rotates about a JT9 axis. The jaw members 104a and 104b pivot about the JT11 axis to open and close.

### Configuration of Arm Operation Unit

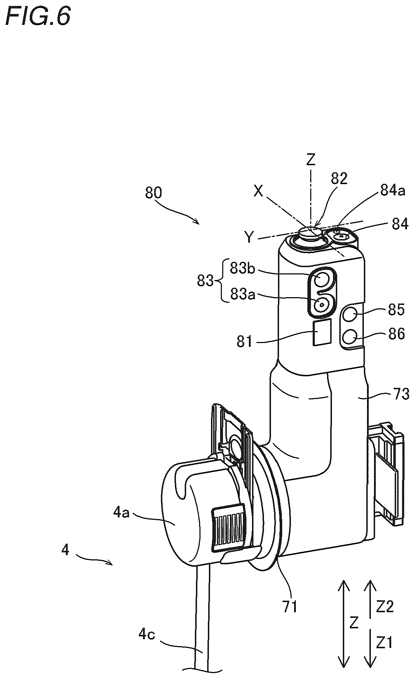

As shown in FIG. 6, the arm operation unit 80 is attached to the robot arm 60 to operate the robot arm 60. Specifically, the arm operation unit 80 is attached to the second link 73.

The arm operation unit 80 includes an enable switch 81, a joystick 82, and linear switches 83, a mode switching button 84, a mode indicator 84a, a pivot button 85, and an adjustment button 86. The enable switch 81 is an example of a second enable switch. The pivot button 85 and the mode switching button 84 are examples of a pivot position setter and a mode switch, respectively.

The enable switch 81 enables or disables movement of the robot arm 60 in response to the joystick 82 and the linear switches 83. When the enable switch 81 is pressed by an operator such as a nurse or an assistant grasping the arm operation unit 80, movement of the surgical instrument 4 by the robot arm 60 is enabled.

The joystick 82 is an operation tool to control movement of the surgical instrument 4 by the robot arm 60. The joystick 82 controls a moving direction and a moving speed of the robot arm 60. The robot arm 60 is moved in accordance with a tilting direction and a tilting angle of the joystick 82.

The linear switches 83 are switches to move the surgical instrument 4 in the Z direction, which is the longitudinal direction of the surgical instrument 4. The linear switches 83 include a linear switch 83a to move the surgical instrument 4 in a direction in which the surgical instrument 4 is inserted into a patient P, and a linear switch 83b to move the surgical instrument 4 in a direction in which the surgical instrument 4 is moved away from the patient P. Both the linear switch 83a and the linear switch 83b are push-button switches.

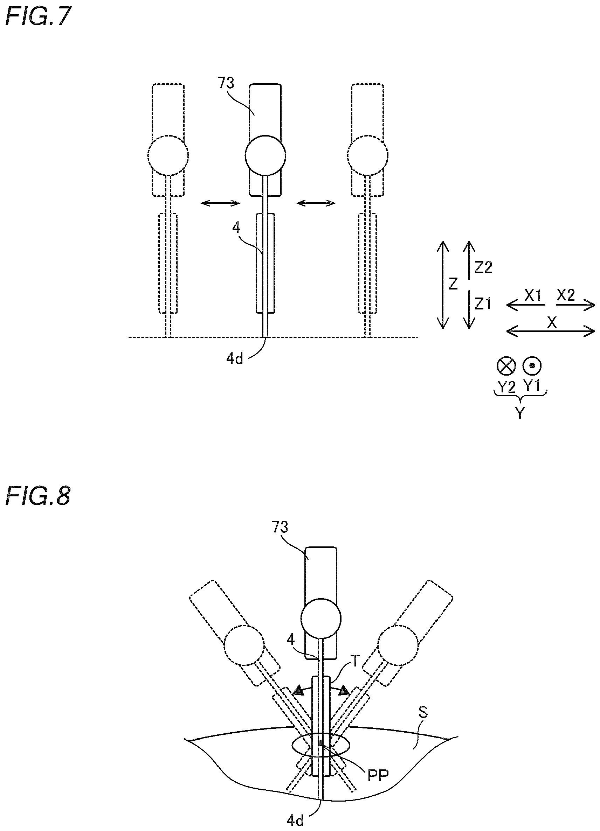

The mode switching button 84 is a push-button switch to switch between a mode for translationally moving the surgical instrument 4 as shown in FIG. 7 and a mode for rotationally moving the surgical instrument 4 as shown in FIG. 8. As shown in FIG. 7, in the mode for translationally moving the robot arm 60, the robot arm 60 is moved such that a distal end 4d of the surgical instrument 4 is moved in an X-Y plane. As shown in FIG. 8, in the mode for rotationally moving the robot arm 60, the robot arm 60 is moved such that the surgical instrument 4 is rotationally moved about a center of the JT11 axis of the pair of forceps 4b as a fulcrum when any pivot position PP is not stored in a storage 32 or a storage 35, and the surgical instrument 4 is rotationally moved about the pivot position PP as a fulcrum when the pivot position PP is stored in the storage 32 and the storage 35. In this case, the surgical instrument 4 is rotationally moved with the shaft 4c of the surgical instrument 4 inserted into a trocar T. The mode switching button 84 is arranged on a Z-direction side surface of the arm operation unit 80. The mode switching button 84 may be other than a push-button switch.

The mode indicator 84a indicates a switched mode. The mode indicator 84a is on to indicate a rotational movement mode and is off to indicate a translational movement mode. Furthermore, the mode indicator 84a also serves as a pivot position indicator that indicates that the pivot position PP has been set. The mode indicator 84a is arranged on the Z-direction side surface of the arm operation unit 80.

The pivot button 85 is a push-button switch to set the pivot position PP that serves as a fulcrum for movement of the surgical instrument 4 attached to the robot arm 60. The pivot button 85 may be other than a push-button switch.

The adjustment button 86 is a button to optimize the position of the robot arm 60. After the pivot position PP for the robot arm 60 to which the endoscope 6 has been attached is set, the positions of the other robot arms 60 and the arm base 50 are optimized when the adjustment button 86 is pressed.

### Remote Control Apparatus

As shown in FIG. 1, the remote control apparatus 2 is arranged inside or outside the operating room, for example. The remote control apparatus 2 includes an operation unit 120 including arms 121 and an operation handle 21, foot pedals 22, a touch panel 23, a monitor 24, a support arm 25, and a support bar 26. The operation unit 120 includes an operation handle for the operator such as a doctor to input a command. The monitor 24 is an example of a display.

The operation unit 120 includes a handle to operate the surgical instrument 4. The operation unit 120 receives an operation amount for the surgical instrument 4. The operation unit 120 includes an operation unit 120L located on the left side as viewed from the operator such as a doctor and operated by the left hand of the operator, and an operation unit 120R located on the right side and operated by the right hand of the operator. The operation unit 120L and the operation unit 120R include an operation handle 21L and an operation handle 21R, respectively.

The monitor 24 is a scope-type display that displays an image captured by the endoscope 6. The support arm 25 supports the monitor 24 so as to align the height of the monitor 24 with the height of the face of the operator such as a doctor. The touch panel 23 is arranged on the support bar 26. The head of the operator is detected by a sensor provided in the vicinity of the monitor 24 such that the surgical robot 1 can be operated by the remote control apparatus 2. The operator operates the operation unit 120 and the foot pedals 22 while visually recognizing an affected area on the monitor 24. Thus, a command is input to the remote control apparatus 2. The command input to the remote control apparatus 2 is transmitted to the surgical robot 1.

### Configuration of Control System

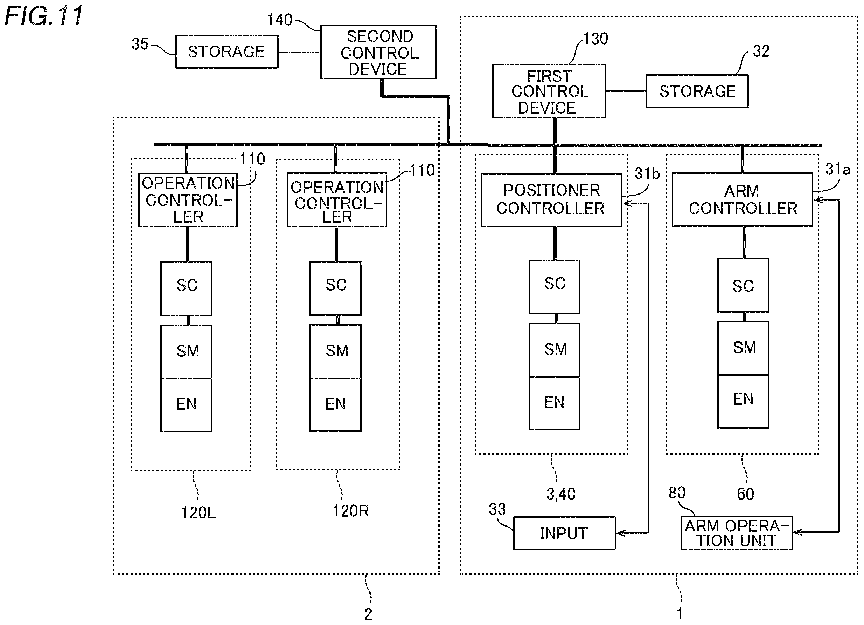

As shown in FIG. 11, the robotic surgical system 100 includes a first control device 130, an arm controller 31a, a positioner controller 31b, operation controllers 110, and a second control device 140. The first control device 130 and the second control device 140 are examples of a control device.

The first control device 130 is accommodated in the medical cart 3 to communicate with the arm controller 31a and the positioner controller 31b, and controls the entire robotic surgical system 100. Specifically, the first control device 130 communicates with the arm controller 31a, the positioner controller 31b, and the operation controllers 110. The first control device 130 controls the arm controller 31a, the positioner controller 31b, and the operation controllers 110. The first control device 130 is connected to the arm controller 31a, the positioner controller 31b, and the operation controllers 110 through a LAN, for example. Although the first control device 130 is shown separately from the medical cart 3 in FIG. 11, FIG. 11 is a diagram for illustrating a control block, and in reality, the first control device 130 is arranged inside the medical cart 3, as shown in FIG. 1.

The arm controller 31a is arranged for each of the plurality of robot arms 60. Although FIG. 11 shows that the arm controller 31a is arranged inside the robot arm 60, FIG. 11 is a diagram for illustrating a control block, and in reality, the arm controller 31a is arranged inside the medical cart 3, as shown in FIG. 1. That is, the same number of arm controllers 31a as the plurality of robot arms 60 are arranged inside the medical cart 3.

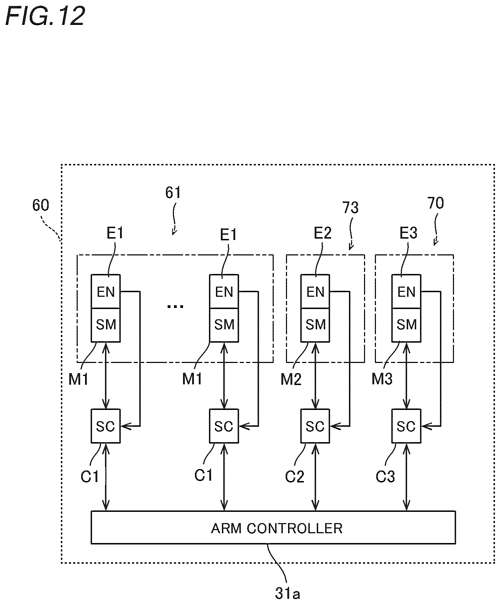

As shown in FIG. 12, the arm portion 61 includes a plurality of servomotors M1, encoders E1, and speed reducers so as to correspond to a plurality of joints 64. The encoders E1 detect rotation angles of the servomotors M1. The speed reducers slow down rotation of the servomotors M1 to increase the torques. Servo controllers C1 are provided to control the servomotors M1. The encoders E1 that detect the rotation angles of the servomotors M1 are electrically connected to the servo controllers C1.

Servomotors M2 to rotate driven members provided in the driven unit 4a of the surgical instrument 4, encoders E2, and speed reducers are arranged in the second link 73. The encoders E2 detect rotation angles of the servomotors M2. The speed reducers slow down rotation of the servomotors M2 to increase the torques. Servo controllers C2 are provided to control the servomotors M2 to drive the surgical instrument 4. The encoders E2 that detect the rotation angles of the servomotors M2 are electrically connected to the servo controllers C2. A plurality of servomotors M2, a plurality of encoders E2, and a plurality of servo controllers C2 are arranged.

The translation mechanism 70 includes a servomotor M3 to translationally move the surgical instrument 4, an encoder E3, and a speed reducer. The encoder E3 detects a rotation angle of the servomotor M3. The speed reducer slows down rotation of the servomotor M3 to increase the torque. A servo controller C3 is provided to control the servomotor M3 to translationally move the surgical instrument 4. The encoder E3 that detects the rotation angle of the servomotor M3 is electrically connected to the servo controller C3.

As shown in FIG. 11, the first control device 130 controls the robot arm 60 based on an operation received by the arm operation unit 80. For example, the first control device 130 controls the robot arm 60 based on an operation received by the joystick 82 of the arm operation unit 80. Specifically, the arm controller 31a outputs an input signal input from the joystick 82 to the first control device 130. The first control device 130 generates position commands based on the received input signal and the rotation angles detected by the encoders E1, and outputs the position commands to the servo controllers C1 via the arm controller 31a. The servo controllers C1 generate current commands based on the position commands input from the arm controller 31a and the rotation angles detected by the encoders E1, and output the current commands to the servomotors M1. Thus, the robot arm 60 is moved according to an operation command input to the joystick 82.

The first control device 130 controls the robot arm 60 based on an input signal from either linear switch 83 of the arm operation unit 80. Specifically, the arm controller 31a outputs the input signal input from the linear switch 83 to the first control device 130. The first control device 130 generates a position command(s) based on the received input signal and the rotation angle(s) detected by the encoders E1 or the encoder E3, and outputs the position command(s) to the servo controllers C1 or the servo controller C3 via the arm controller 31a. The servo controllers C1 or the servo controller C3 generates a current command(s) based on the position command(s) input from the arm controller 31a and the rotation angle(s) detected by the encoders E1 or the encoder E3, and outputs the current command(s) to the servomotors M1 or the servomotor M3. Thus, the robot arm 60 is moved according to an operation command input to the linear switch 83.

The positioner controller 31b is arranged in the medical cart 3. The positioner controller 31b controls the positioner 40 and the medical cart 3. Servomotors SM, encoders EN, speed reducers, and servo controllers SC are provided in the positioner 40 so as to correspond to a plurality of joints 43 of the positioner 40. Servomotors SM that drive a plurality of front wheels of the medical cart 3, encoders EN, speed reducers, servo controllers SC, and brakes are arranged in the medical cart 3.

The operation controllers 110 control the operation unit 120. The operation controllers 110 are provided so as to correspond to the operation unit 120L and the operation unit 120R, respectively. Servomotors SM, encoders EN, speed reducers, and servo controllers SC are provided in the operation unit 120 so as to correspond to a plurality of joints of the operation unit 120. The servo controllers SC configured to control the servomotors SM of the operation unit 120 is provided in the main body of the remote control apparatus 2 adjacent to the operation controller 110.

The second control device 140 is arranged separately from the first control device 130.

### Pivot Position Setting

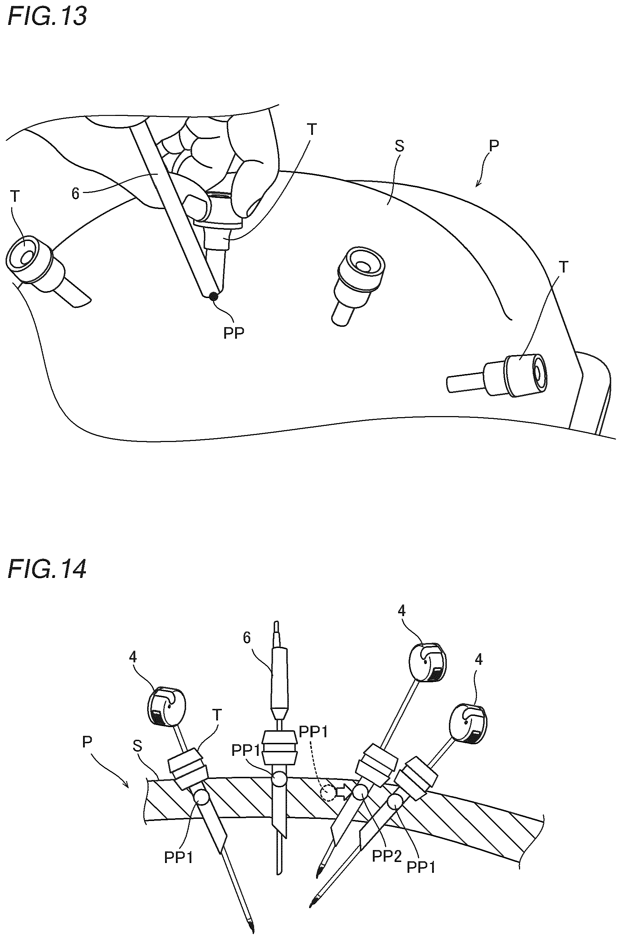

Pivot position PP setting is described. When the pivot button 85 is operated in a state in which a distal end of the endoscope 6 or a pivot position setting instrument 7 attached to the distal end side of the robot arm 60 is moved to a position corresponding to an insertion position of the trocar T inserted into the body surface S of the patient P as shown in FIG. 13 by operating the robot arm 60 using the arm operation unit 80, the second control device 140 causes the storage 35 to store the pivot position PP. The pivot position PP is stored as one coordinate point, and in pivot position PP setting, the direction of the surgical instrument 4 is not set. Operating the pivot button 85 refers to pressing the pivot button 85.

### Pivot Position Change

In the following description, the pivot position stored in the storage 35 by the second control device 140 in a state in which the distal end of the endoscope 6 or the pivot position setting instrument 7 is moved to the position corresponding to the insertion position of the trocar T is defined as a first pivot position PP1. In the present embodiment, as shown in FIG. 14, the second control device 140 makes a transition to a pivot position change mode for changing the first pivot position PP1 stored in the storage 35 based on the pivot button 85 being operated in a state in which the surgical instrument 4 is inserted into the body of the patient P, and causes the storage 35 to store, as a second pivot position PP2, the pivot position after the surgical instrument 4 is moved, when the pivot button 85 is operated after the surgical instrument 4 is moved by the arm operation unit 80 in the pivot position change mode. Specifically, the second control device 140 causes the storage 35 to store, as the second pivot position PP2 after change, the pivot position after the surgical instrument 4 is moved, when the pivot button 85 is operated after the surgical instrument 4 is moved by at least one of the joystick 82 or the linear switch 83 in the pivot position change mode. The first pivot position PP1 can be changed for both the robot arm 60 to which the endoscope 6 is attached and the robot arms 60 to which the surgical instruments 4 other than the endoscope 6 are attached.

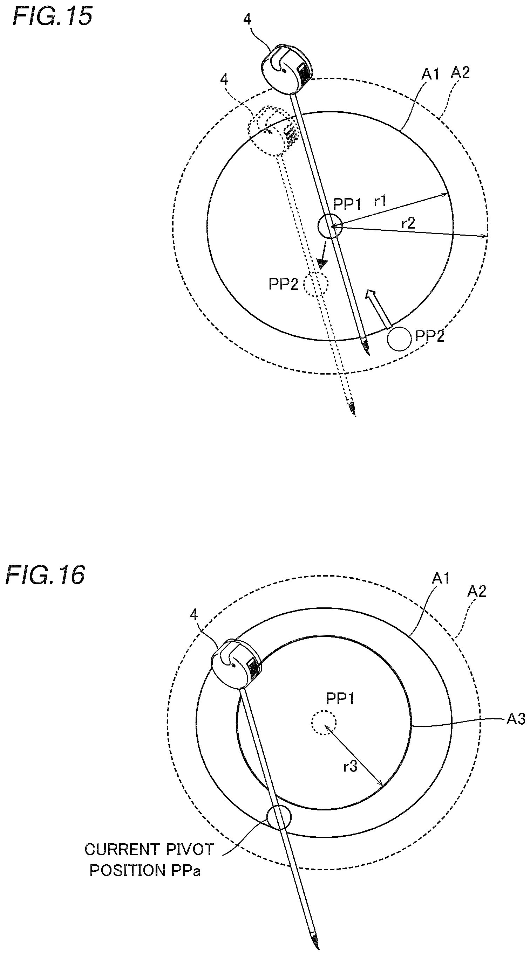

In the present embodiment, as shown in FIG. 15, the second control device 140 receives a change in the first pivot position PP1 within a pivot correction possible range A1 set based on the first pivot position PP1 stored in the storage 35. The pivot correction possible range A1 is inside a sphere having a radius r1 centered on the first pivot position PP1 stored in the storage 35, and is calculated by the first control device 130. Furthermore, the second control device 140 causes the storage 35 to store, as a third pivot position PP3, the pivot position after the surgical instrument 4 is moved within the pivot correction possible range A1 set based on the first pivot position PP1, when a transition is made to the pivot position change mode with the second pivot position PP2 stored in the storage 35. In other words, even when the second pivot position PP2 is changed after the first pivot position PP1 is changed, the pivot correction possible range A1 is inside the sphere having the radius r1 centered on the first pivot position PP1 initially stored in the storage 35.

In the present embodiment, the second control device 140 does not receive a transition to the pivot position change mode when the second pivot position PP2 is outside a pivot correction start possible range A2 that is larger than the pivot correction possible range A1. The pivot correction start possible range A2 is inside a sphere having a radius r2 centered on the first pivot position PP1 stored in the storage 35. The radius r2 is larger than the radius r1. The second control device 140 monitors whether or not the second pivot position PP2 is outside the pivot correction start possible range A2. The second control device 140 transmits to the first control device 130 that the second pivot position PP2 is outside the pivot correction start possible range A2.

In the present embodiment, the second control device 140 receives an operation to move the surgical instrument 4 only in a direction toward the first pivot position PP1 through the arm operation unit 80 when the second pivot position PP2 is located between the pivot correction possible range A1 and the pivot correction start possible range A2. The second control device 140 receives not only an operation to move the surgical instrument 4 in a direction linearly toward the first pivot position PP1, but also an operation to move the surgical instrument 4 in a direction toward the center of the first pivot position PP1 in a detour. When the surgical instrument 4 is about to be moved in a direction away from the first pivot position PP1, the first control device 130 performs a control to display a message calling for attention on the monitor 24 of the remote control apparatus 2, and issue an audible alarm.

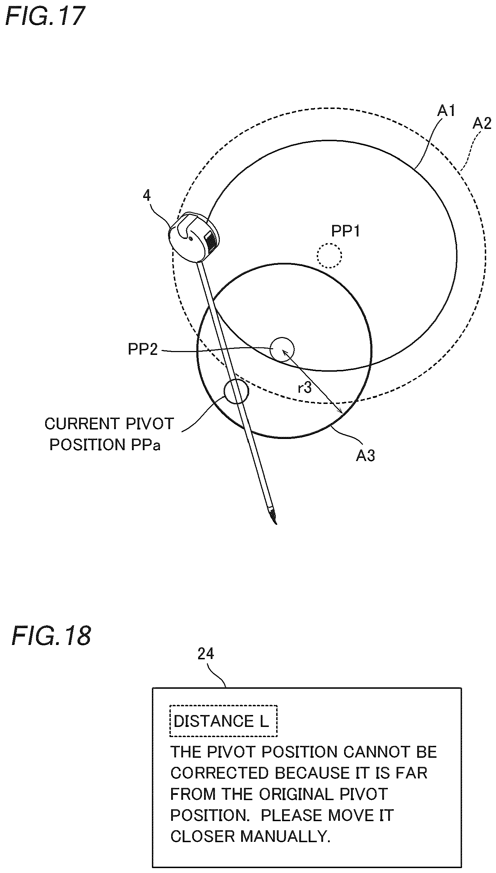

In the present embodiment, as shown in FIG. 18, when the second pivot position PP2 is outside the pivot correction start possible range A2, the first control device 130 causes the monitor 24 of the remote control apparatus 2 to display a message indicating that the second pivot position PP2 is outside the pivot correction start possible range A2. For example, a message is displayed on the monitor 24 stating that the pivot position cannot be corrected because it is far from the original pivot position, and that the pivot position must be moved closer manually.

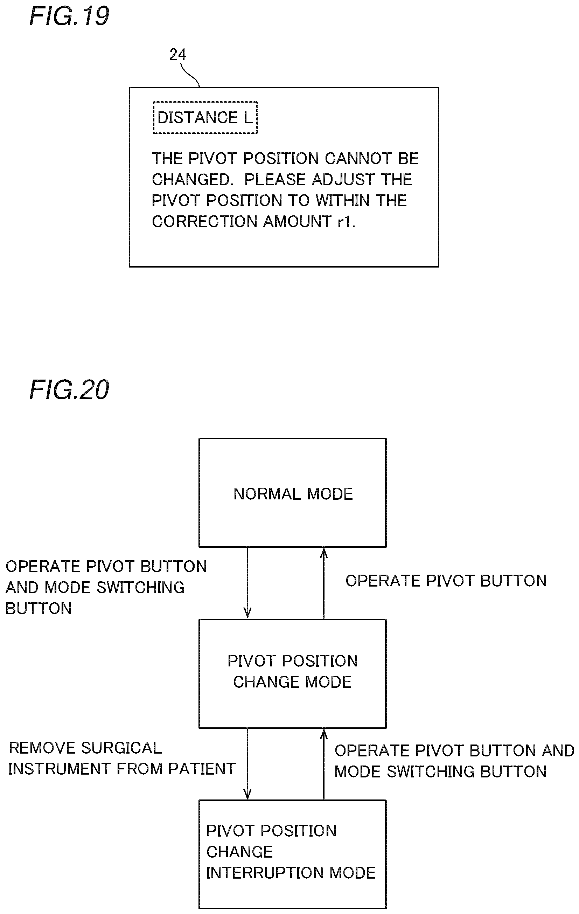

In the present embodiment, as shown in FIG. 19, when the second pivot position PP2 is located between the pivot correction possible range A1 and the pivot correction start possible range A2, the first control device 130 causes the monitor 24 to display a message prompting the operator to adjust the second pivot position PP2 to within the pivot correction possible range A1 when the operator attempts to set the second pivot position PP2 by pressing the pivot button 85. For example, a message is displayed on the monitor 24 stating that the pivot position cannot be changed, and that the pivot position must be adjusted to within a correction amount r1.

In the present embodiment, as shown in FIG. 18, the first control device 130 causes the monitor 24 to display a distance L from the first pivot position PP1 stored in the storage 35 to the second pivot position PP2. The distance L is displayed in real time as the second pivot position PP2 to be changed moves.

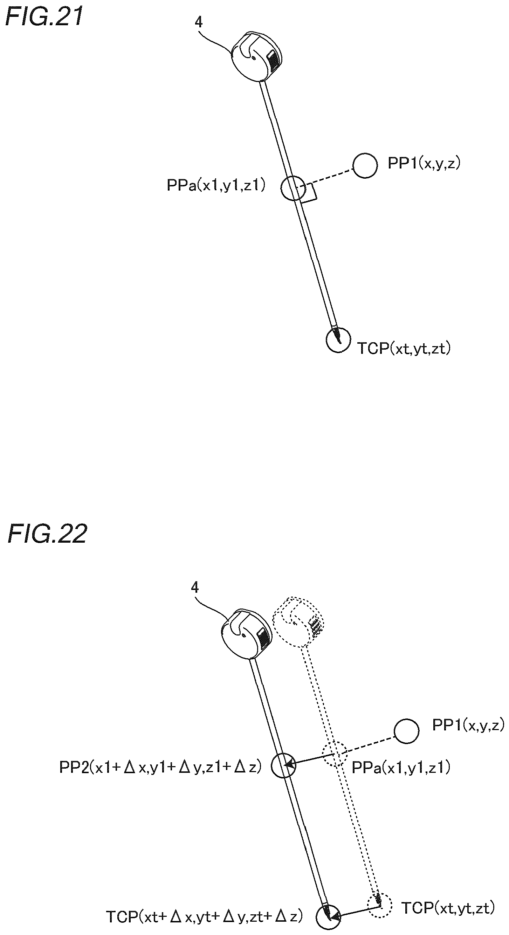

In the present embodiment, as shown in FIG. 16, the second control device 140 causes the monitor 24 to display that there is an abnormality when a deviation between a current pivot position PPa on the surgical instrument 4 and the first pivot position PP1 is not within a pivot deviation monitoring range A3. The second control device 140 monitors whether or not the deviation between the current pivot position PPa on the surgical instrument 4 and the first pivot position PP1 is within the pivot deviation monitoring range A3. The second control device 140 transmits to the first control device 130 that the deviation between the surgical instrument 4 and the first pivot position PP1 is outside the pivot deviation monitoring range A3. The pivot deviation monitoring range A3 is inside a sphere having a radius r3. The current pivot position PPa on the surgical instrument 4 refers to a temporary pivot position PPa shown in FIG. 21, obtained by drawing a perpendicular line from the first pivot position PP1 stored in the storage 35 to the shaft 4c of the surgical instrument 4. Furthermore, the current pivot position PPa is calculated from the detected values of the encoders EN arranged in the positioner 40 and the encoders E1 and E3 arranged in the robot arm 60.

In the present embodiment, as shown in FIG. 16, the second control device 140 notifies the operator such as a doctor of an abnormality when the deviation between the current pivot position PPa and the first pivot position PP1 is not within the pivot deviation monitoring range A3 in a normal mode. The normal mode refers to a mode separated from the pivot position change mode and a pivot position change interruption mode described below. Although in FIG. 16, the pivot correction possible range A1, the pivot correction start possible range A2, and the pivot deviation monitoring range A3 are illustrated, in the normal mode, two parameters are present for control: the pivot correction possible range A1 and the pivot deviation monitoring range A3. Furthermore, when a transition is made to the pivot position change mode or the pivot position change interruption mode, two parameters are present for control: the pivot correction possible range A1 and the pivot correction start possible range A2. In terms of control, the pivot correction start possible range A2 and the pivot deviation monitoring range A3 are defined by the same parameters. The radius r1 of the pivot correction possible range A1 and the radius r2 of the pivot correction start possible range A2 are larger than the radius r3 of the pivot deviation monitoring range A3.

As shown in FIG. 17, after the first pivot position PP1 stored in the storage 35 is changed to the second pivot position PP2, the pivot deviation monitoring range A3 is changed to a pivot deviation monitoring range A3 having a radius r3 centered on the second pivot position PP2. In FIG. 17, the current pivot position PPa is within the pivot deviation monitoring range A3, and thus the operator such as a doctor is not notified of an abnormality.

In the present embodiment, the second control device 140 transitions to the pivot position change mode when both the pivot button 85 and the mode switching button 84 are operated. Specifically, the second control device 140 receives an operation to change the first pivot position PP1 when all of the following conditions are satisfied. Condition 1 is that the surgical instrument 4 is attached to the robot arm 60, the first pivot position PP1 of which is to be changed. Condition 2 is that storage of the first pivot position PP1 has been completed. Condition 3 is that both the pivot button 85 and the mode switching button 84 are operated. Condition 4 is that the endoscope 6 is attached to the robot arm 60. Condition 5 is that the position of the robot arm 60 is not being moved to a replacement position for replacing the endoscope 6. Condition 6 is that the position of the robot arm 60 is not being moved to a replacement position for replacing the surgical instrument 4 other than the endoscope 6. Condition 7 is that the patient is not in a state in which it is possible to start surgery using the surgical instrument 4. Condition 8 is that the enable switch 81 is not operated. Condition 9 is that the second pivot position PP2 to be changed is within the pivot correction start possible range A2 that is larger than the pivot correction possible range A1 set based on the first pivot position PP1 initially stored in the storage 35. Furthermore, moving the position of the robot arm 60 to the replacement position for replacing the surgical instrument 4 refers to moving the robot arm 60 to the near side by a predetermined distance from the position of the robot arm 60 before replacement when the surgical instrument 4 is replaced and the surgical instrument 4 is inserted into the patient P again. This operation is called a guide tool change. A state in which surgery using the surgical instrument 4 can be started refers to a state in which the surgical instrument 4 can be operated by the arm operation unit 80 of the remote control apparatus 2. This state is called a following-enabled state. Both the pivot button 85 and the mode switching button 84 being operated means that both the pivot button 85 and the mode switching button 84 have been pressed for a predetermined period of time or longer. The enable switch 81 being operated means that the enable switch 81 is being pressed.

In the present embodiment, as shown in FIG. 20, when both the pivot button 85 and the mode switching button 84 are operated, the second control device 140 makes a transition from the normal mode to the pivot position change mode for changing the first pivot position PP1. The first control device 130 transmits a pivot position correction switching enable signal to the second control device 140 when Conditions 1, 3, 5, 6, 7, 8, and 9 are satisfied. Thus, the second control device 140 makes a transition to the pivot position change mode.

In the present embodiment, when the pivot button 85 is operated in the pivot position change mode, the second control device 140 causes the storage 35 to store the second pivot position PP2, and then terminates the pivot position change mode and makes a transition to the normal mode. When the surgical instrument 4 is removed from the patient P in the pivot position change mode, the second control device 140 interrupts the pivot position change mode and makes a transition to the pivot position change interruption mode. Although not shown in FIG. 20, when the robotic surgical system 100 is restarted in the pivot position change mode, the second control device 140 terminates the pivot position change mode and makes a transition to the normal mode. The normal mode refers to a state in which the surgical instrument 4 can be operated by the arm operation unit 80 of the remote control apparatus 2. In the pivot position change interruption mode, the surgical instrument 4 attached to the robot arm 60 may be replaced.

In the present embodiment, the second control device 140 makes a transition to the pivot position change mode when the following conditions are satisfied in the pivot position change interruption mode: the surgical instrument 4 is attached to the robot arm 60, and both the mode switching button 84 and the pivot button 85 are operated. Specifically, the second control device 140 makes a transition from the pivot position change interruption mode to the pivot position change mode when all of the following conditions are satisfied. Condition 11 is that the surgical instrument 4 is attached to the robot arm 60, the first pivot position PP1 of which is to be changed. Condition 12 is that both the mode switching button 84 and the pivot button 85 are operated. Condition 13 is that the position of the robot arm 60 is not being moved to a replacement position for replacing the endoscope 6 or the surgical instrument 4. Condition 14 is that the patient is not in a state in which it is possible to start surgery using the surgical instrument 4. Condition 15 is that the enable switch 81 is not operated. Condition 16 is that the second pivot position PP2 to be changed is within the pivot correction start possible range A2 that is larger than the pivot correction possible range A1 set based on the first pivot position PP1 stored in the storage 35. When the surgical instrument 4 attached to the robot arm 60 is replaced in the pivot position change interruption mode, the second control device 140 make a transition from the pivot position change interruption mode to the pivot position change mode when both the mode switching button 84 and the pivot button 85 are operated after the guide tool change operation is performed.

In the pivot position change mode, the first control device 130 causes the monitor 24 of the remote control apparatus 2 to display a message stating that the pivot is being corrected and that the pivot button should be pressed after the correction is completed. When the first pivot position PP1 is about to be corrected beyond the pivot correction possible range A1 in the pivot position change mode, the first control device 130 causes the monitor 24 to display a message stating that a correction cannot be made in this direction and that the pivot position should be moved in the opposite direction. The first control device 130 causes the monitor 24 to display a message stating that in the pivot position change interruption mode, the pivot correction will be resumed after the guide tool change operation is completed and that both the pivot button 85 and the mode switching button 84 should be pressed.

### Method for Calculating Pivot Position

A method for calculating the pivot position PP is now described. In the following, a case is described in which the first pivot position PP1 stored in the storage 35 and the actual first pivot position PP1 on the surgical instrument 4 are deviated from each other, but the following calculation method is the same even when the first pivot position PP1 is not deviated. As shown in FIG. 21, the coordinates of the first pivot position PP1 stored in the storage 35 are (x, y, z). Furthermore, the center of a clevis of the surgical instrument 4 is called a TCP.

When a transition is made to the pivot position change mode, the second control device 140 sets a position obtained by drawing a perpendicular line from the first pivot position PP1 stored in the storage 35 to the shaft 4c of the surgical instrument 4 as the temporary pivot position PPa. The storage 35 stores the coordinates (x1, y1, z1) of the temporary pivot position PPa. Furthermore, when a transition is made to the pivot position change mode, the second control device 140 sets the position of the TCP based on the posture of the surgical robot 1 calculated from the values of the encoders mounted on the respective axes of the positioner 40 and the robot arm 60. The storage 35 stores the coordinates (xt, yt, zt) of the TCP.

As shown in FIG. 22, when the first pivot position PP1 is moved by the joystick 82 or the linear switch 83, the second control device 140 causes the storage 35 to store the coordinates (xt+Δx, yt+Δy, zt+Δz) of the TCP after the movement. Then, the movement amount (Δx, Δy, Δz) of the TCP is calculated. The second control device 140 causes the storage 35 to store the coordinates (x1+Δx, y1+Δy, z1+Δz) obtained by adding the calculated movement amount (Δx, Δy, Δz) to the coordinates (x1, y1, z1) of the temporary pivot position PPa as the coordinates of the second pivot position PP2 after change. Furthermore, a straight line connecting the first pivot position PP1 before the movement to the TCP before the movement is parallel to a straight line connecting the second pivot position PP2 after the movement to the TCP after the movement.

### Control Method for Robotic Surgical System

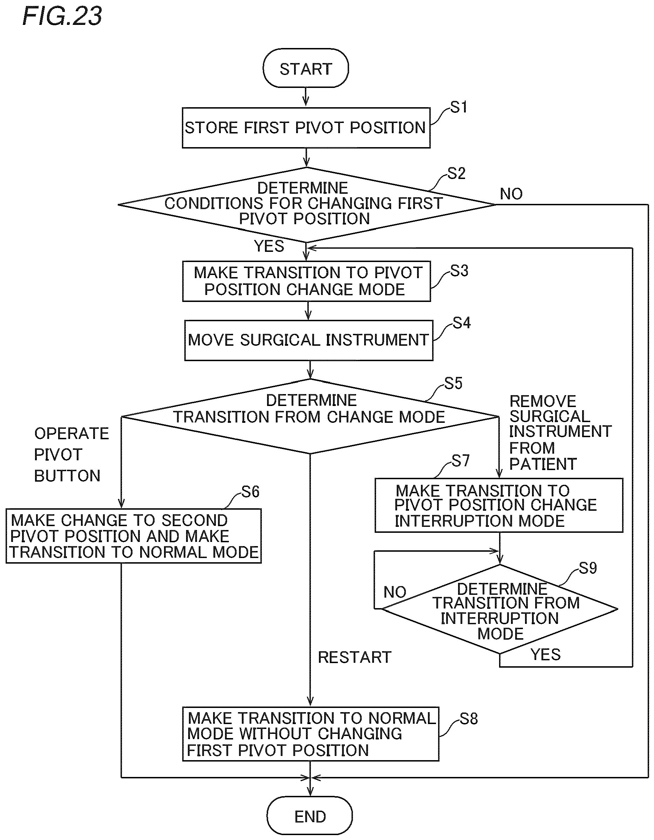

A control method for the robotic surgical system 100 is now described. As shown in FIG. 23, in step S1, when the pivot button 85 is operated in a state in which the distal end of the endoscope 6 or the pivot position setting instrument 7 attached to the distal end side of the robot arm 60 is moved to the position corresponding to the insertion position of the trocar T inserted into the body surface S of the patient P, the second control device 140 causes the storage 35 to store the first pivot position PP1.

In step S2, the second control device 140 determines whether or not all of Conditions 1 to 9 described above are satisfied. The first control device 130 transmits the pivot position correction switching enable signal to the second control device 140 when Condition 1, Condition 3, Condition 5, Condition 6, Condition 7, and Condition 9 are satisfied. Thus, the second control device 140 makes a transition to the pivot position change mode in step S3.

In step S4, the second control device 140 receives an operation to move the surgical instrument 4 through the joystick 82 and the linear switch 83.

In step S5, a transition from the pivot position change mode is determined. When the pivot button 85 is operated, in step S6, the second control device 140 causes the storage 35 to store, as the second pivot position PP2, the pivot position after the surgical instrument 4 is moved. The second control device 140 makes a transition from the pivot position change mode to the normal mode.

When the surgical instrument 4 is removed from the patient P, in step S7, the second control device 140 interrupts the pivot position change mode and makes a transition to the pivot position change interruption mode.

When the robotic surgical system 100 is restarted, the second control device 140 terminates the pivot position change mode and makes a transition to the normal mode in step S8.

In step S9, the second control device 140 determines whether or not all of Conditions 11 to 16 are satisfied. In a case of YES in step S9, the process advances to step S3 when both the pivot button 85 and the mode switching button 84 are operated, and the second control device 140 makes a transition from the pivot position change interruption mode to the pivot position change mode.

### Advantages of Present Embodiment

The second control device 140 is configured or programmed to cause the storage 35 to store the first pivot position PP1 that serves as the fulcrum for movement of the surgical instrument 4 attached to the robot arm 60 based on the pivot button 85 being operated. Accordingly, the second control device 140 causes the storage 35 to store the first pivot position PP1 by software, and thus it is not necessary to use a manipulator arm with a structurally defined first pivot position or to arrange a mechanism on a manipulator arm to support a cannula in order to set the first pivot position PP1. Consequently, no mechanism for supporting the cannula is arranged, and thus work in the vicinity of the body surface S of the patient P can be easily performed during surgery. Furthermore, the second control device 140 is configured or programmed to make a transition to the pivot position change mode for changing the first pivot position PP1 stored in the storage 35 based on the pivot button 85 being operated in a state in which the surgical instrument 4 is inserted into the body of the patient P, and to cause the storage 35 to store, as the second pivot position PP2, the pivot position after the surgical instrument 4 is moved, when the pivot button 85 is operated after the surgical instrument 4 is moved by the arm operation unit 80 in the pivot position change mode. Thus, the second control device 140 changes the first pivot position PP1 by software, and thus the first pivot position PP1 that serves as the fulcrum for movement of the surgical instrument 4 can be easily changed. Consequently, an operation to avoid interference of the robot arm 60 and an operation to expand the range of motion of the surgical instrument 4 within the patient P can be easily performed.

The second control device 140 is configured or programmed to cause the storage 35 to store the second pivot position PP2 within the pivot correction possible range A1 set based on the first pivot position PP1. Thus, the range of change of the second pivot position PP2 is limited, and thus it is possible to reduce or prevent a change of the second pivot position PP2 to a position that is too far away.

The second control device 140 is configured or programmed to cause the storage 35 to store, as the third pivot position PP3, the pivot position after the surgical instrument 4 is moved within the pivot correction possible range A1 set based on the first pivot position PP1, when a transition is made to the pivot position change mode with the second pivot position PP2 stored in the storage 35. Accordingly, even when the first pivot position PP1 is changed a plurality of times, it is possible to reduce or prevent a change of the first pivot position PP1 to a position that is too far away.

The second control device 140 is configured or programmed to not receive a transition to the pivot position change mode when the second pivot position PP2 is outside the pivot correction start possible range A2 that is larger than the pivot correction possible range A1, and to receive an operation to move the surgical instrument 4 only in the direction toward the first pivot position PP1 through the arm operation unit 80 when the second pivot position PP2 is located between the pivot correction possible range A1 and the pivot correction start possible range A2. Accordingly, the pivot correction start possible range A2 is larger than the pivot correction possible range A1, and thus it is possible to reduce or prevent limitation of a range in which pivot correction can be started to a small range. Furthermore, when the second pivot position PP2 is located between the pivot correction possible range A1 and the pivot correction start possible range A2, the operation to move the surgical instrument 4 only in the direction toward the first pivot position PP1 is received through the arm operation unit 80, and thus the operator can be prompted to set the second pivot position PP2 within the pivot correction possible range A1.

The first control device 130 is configured or programmed to cause the monitor 24 to display the message indicating that the second pivot position PP2 is outside the pivot correction start possible range A2 when the second pivot position PP2 is outside the pivot correction start possible range A2, and to cause the monitor 24 to display the message prompting the operator to adjust the second pivot position PP2 to within the pivot correction possible range A1 when the second pivot position PP2 is located between the pivot correction possible range A1 and the pivot correction start possible range A2. Accordingly, the operator can easily recognize that the second pivot position PP2 is outside the pivot correction start possible range A2, and that the second pivot position PP2 is located between the pivot correction possible range A1 and the pivot correction start possible range A2.

The first control device 130 is configured or programmed to cause the monitor 24 to display the distance L from the first pivot position PP1 to the second pivot position PP2. Accordingly, the operator can easily recognize the distance L by visually checking the monitor 24.

The second control device 140 is configured or programmed to cause the monitor 24 to display that there is an abnormality when the deviation between the current pivot position PPa on the surgical instrument 4 and the first pivot position PP1 is not within the pivot deviation monitoring range A3. Accordingly, the operator can easily recognize that the current pivot position PPa on the surgical instrument 4 and the first pivot position PP1 are excessively deviated from each other.

The second control device 140 is configured or programmed to notify the operator of an abnormality when the deviation between the current pivot position PPa and the first pivot position PP1 is not within the pivot deviation monitoring range A3 in the normal mode. Accordingly, in the normal mode, the current pivot position PPa is constantly monitored such that an excessive deviation between the current pivot position PPa and the first pivot position PP1 can be reduced or prevented.

The second control device 140 is configured or programmed to make a transition to the pivot position change mode when both the pivot button 85 and the mode switching button 84 are operated. Accordingly, a transition to the pivot position change mode due to an erroneous operation on one of the pivot button 85 and the mode switching button 84 can be reduced or prevented.

The second control device 140 is configured or programmed to cause the storage 35 to store, as the second pivot position PP2, the pivot position after the surgical instrument 4 is moved, when the pivot button 85 is operated after the surgical instrument 4 is moved by at least one of the joystick 82 or the linear switch 83 in the pivot position change mode. Accordingly, the surgical instrument 4 can be easily moved by at least one of the joystick 82 or the linear switch 83, and thus the first pivot position PP1 can be easily changed to the second pivot position PP2.

The second control device 140 is configured or programmed to cause the storage 35 to store the second pivot position PP2, and then terminate the pivot position change mode and make a transition to the normal mode when the pivot button 85 is operated in the pivot position change mode. Furthermore, the second control device 140 is configured or programmed to interrupt the pivot position change mode and make a transition to the pivot position change interruption mode when the surgical instrument 4 is removed from the patient P in the pivot position change mode. Moreover, the second control device 140 is configured or programmed to terminate the pivot position change mode and make a transition to the normal mode when the robotic surgical system 100 is restarted in the pivot position change mode. Accordingly, the first pivot position PP1 can be reliably changed to the second pivot position PP2 by operating the pivot button 85 based on the operator's intention. Furthermore, a change in the first pivot position PP1 can be reduced or prevented when the operator does not intend to change the first pivot position PP1, such as when the surgical instrument 4 is removed or the robotic surgical system 100 is restarted.

The second control device 140 is configured or programmed to make a transition to the pivot position change mode when the following conditions are satisfied: the surgical instrument 4 is attached to the robot arm 60, and both the mode switching button 84 and the pivot button 85 are operated. Accordingly, it is possible to make a transition to the pivot position change mode again, and thus when the surgical instrument 4 is removed from the patient P and the surgical instrument 4 is replaced, for example, the first pivot position PP1 can be changed without performing an initial setting of the first pivot position PP1.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the pivot correction start possible range A2 is larger than the pivot correction possible range A1 has been shown in the aforementioned embodiment, the present disclosure is not limited to this. For example, the size of the pivot correction start possible range A2 and the size of the pivot correction possible range A1 may be the same as each other.

While the example in which the message indicating that the second pivot position PP2 is outside the pivot correction start possible range A2, the message prompting the operator to adjust the second pivot position PP2 to within the pivot correction possible range A1, etc. are displayed on the monitor 24 has been shown in the aforementioned embodiment, the present disclosure is not limited to this. For example, these messages may be displayed on the display 33a in addition to the monitor 24. Alternatively, these messages may be announced by voice.

While the example in which the second control device 140 makes a transition to the pivot position change mode when all of Conditions 1 to 9 described above are satisfied has been shown in the aforementioned embodiment, the present disclosure is not limited to this. For example, the second control device 140 may make a transition to the pivot position change mode when some but not all of Conditions 1 to 9 described above are satisfied.

While the example in which the second control device 140 makes a transition from the pivot position change mode to the pivot position change mode when all of Conditions 11 to 16 described above are satisfied has been shown in the aforementioned embodiment, the present disclosure is not limited to this. For example, the second control device 140 may make a transition to the pivot position change mode when some but not all of Conditions 11 to 16 described above are satisfied.

While the example in which two control devices, the first control device 130 and the second control device 140, are arranged has been shown in the aforementioned embodiment, the present disclosure is not limited to this. For example, one control device may perform the controls in the aforementioned embodiment.

While the example in which four robot arms 60 are provided has been shown in the aforementioned embodiment, the present disclosure is not limited to this. In the present disclosure, the number of robot arms 60 may be any other number as long as at least one robot arm is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor. Description of Reference Numerals

4: surgical instrument
6: endoscope
24: monitor (display)
35: storage
50: arm base
60: robot arm
60a, 60b, 60d: robot arm (first robot arm)
60c: robot arm (second robot arm)
80: arm operation unit (operation unit)
82: joystick
83: linear switch
84: mode switching button (mode switch)
85: pivot button (pivot position setter)
100: robotic surgical system
130: first control device (control device)
140: second control device (control device)
A1: pivot correction possible range
A2: pivot correction start possible range
A3: pivot deviation monitoring range
L: distance
P: patient
PP: pivot position
PP1: first pivot position
PP2: second pivot position
PP3: third pivot position

## Claims

1. A robotic surgical system comprising:
a robot arm having a distal end to which a surgical instrument is attached;
an operation unit attached to the robot arm to operate the robot arm;
a pivot position setter to set a pivot position that serves as a fulcrum for movement of the surgical instrument attached to the robot arm;
a storage; and
a control device; wherein
the control device is configured or programmed to:
cause the storage to store a first pivot position based on the pivot position setter being operated;
make a transition to a pivot position change mode for changing the first pivot position stored in the storage based on the pivot position setter being operated in a state in which the surgical instrument is inserted into a body of a patient; and
cause the storage to store, as a second pivot position, the pivot position after the surgical instrument is moved, when the pivot position setter is operated after the surgical instrument is moved by the operation unit in the pivot position change mode.

2. The robotic surgical system according to claim 1, wherein the control device is configured or programmed to cause the storage to store the second pivot position within a pivot correction possible range set based on the first pivot position.

3. The robotic surgical system according to claim 2, wherein the control device is configured or programmed to cause the storage to store, as a third pivot position, the pivot position after the surgical instrument is moved within the pivot correction possible range set based on the first pivot position, when a transition is made to the pivot position change mode with the second pivot position stored in the storage.

4. The robotic surgical system according to claim 3, wherein
the control device is configured or programmed to:
not receive a transition to the pivot position change mode when the second pivot position is outside a pivot correction start possible range that is larger than the pivot correction possible range; and
receive an operation to move the surgical instrument through the operation unit only in a direction toward the first pivot position when the second pivot position is located between the pivot correction possible range and the pivot correction start possible range.

5. The robotic surgical system according to claim 4, further comprising:
a display; wherein
the control device is configured or programmed to:
cause the display to display a message indicating that the second pivot position is outside the pivot correction start possible range when the second pivot position is outside the pivot correction start possible range; and
cause the display to display a message prompting an operator to adjust the pivot position to within the pivot correction possible range when the second pivot position is located between the pivot correction possible range and the pivot correction start possible range.

6. The robotic surgical system according to claim 1, further comprising:
a display; wherein
the control device is configured or programmed to cause the display to display a distance from the first pivot position to the second pivot position.

7. The robotic surgical system according to claim 1, wherein the control device is configured or programmed to notify an operator of an abnormality when a deviation between a current pivot position on the surgical instrument and the first pivot position is not within a pivot deviation monitoring range.

8. The robotic surgical system according to claim 7, wherein the control device is configured or programmed to notify the operator of the abnormality when the deviation between the current pivot position and the first pivot position is not within the pivot deviation monitoring range in a normal mode.

9. The robotic surgical system according to claim 1, wherein
the robot arm is a first robot arm; and
the robotic surgical system further comprises:
a second robot arm to which an endoscope is attached; and
an arm base to which the first and second robot arms are attached.

10. The robotic surgical system according to claim 1, wherein
the operation unit includes a mode switch to switch between a translational movement mode for translationally moving the surgical instrument and a rotational movement mode for rotationally moving the surgical instrument; and
the control device is configured or programmed to make a transition to the pivot position change mode when both the pivot position setter and the mode switch are operated.

11. The robotic surgical system according to claim 1, wherein
the operation unit includes:
a joystick to receive an operation to move a distal end of the surgical instrument along a plane; and
a linear switch to receive an operation to move the surgical instrument along a longitudinal direction of the surgical instrument; and
the control device is configured or programmed to cause the storage to store, as the second pivot position, the pivot position after the surgical instrument is moved, when the pivot position setter is operated after the surgical instrument is moved by at least one of the joystick or the linear switch in the pivot position change mode.

12. The robotic surgical system according to claim 1, wherein
the operation unit includes a mode switch to switch between a translational movement mode for translationally moving the surgical instrument and a rotational movement mode for rotationally moving the surgical instrument; and
the control device is configured or programmed to, in the pivot position change mode:
cause the storage to store the second pivot position, and then terminate the pivot position change mode and make a transition to a normal mode when the pivot position setter is operated;
interrupt the pivot position change mode and make a transition to a pivot position change interruption mode when the surgical instrument is removed from the patient; and
terminate the pivot position change mode and make a transition to the normal mode when the robotic surgical system is restarted.

13. The robotic surgical system according to claim 12, wherein the control device is configured or programmed to make a transition to the pivot position change mode when following conditions are satisfied in the pivot position change interruption mode: the surgical instrument is attached to the robot arm, and both the mode switch and the pivot position setter are operated.

14. A control method for a robotic surgical system, the control method comprising:
causing a storage to store a first pivot position that serves as a fulcrum for movement of a surgical instrument attached to a robot arm based on a pivot position setter being operated;
making a transition to a pivot position change mode for changing the first pivot position stored in the storage based on the pivot position setter being operated in a state in which the surgical instrument is inserted into a body of a patient; and
causing the storage to store, as a second pivot position, a pivot position after the surgical instrument is moved, when the pivot position setter is operated after the surgical instrument is moved by an operation unit in the pivot position change mode.
